# FASCICULE DE BREVET EUROPEEN

(11) **EP 3 886 952 B1**
(45) Date de publication et mention de la délivrance du brevet: **16.11.2022**
(21) Numéro de dépôt: 19835697.4
(22) Date de dépôt: 29.11.2019
(51) Int. Cl.: A61M 15/00

(54) **DISPOSITIF DE DISTRIBUTION DE PRODUIT FLUIDE SYNCHRONISE AVEC L'INHALATION**
VORRICHTUNG ZUR INHALATIONSSYNCHRONISIERTEN AUSGABE EINES FLÜSSIGPRODUKTS
DEVICE FOR INHALATION-SYNCHRONISED DISPENSING OF A FLUID PRODUCT

(30) Priorité: 30.11.2018 FR 1872152
(43) Date de publication de la demande: 06.10.2021
(73) Titulaire: APTAR France SAS, 27110 Le Neubourg (FR)
(72) Inventeur: FABIEN, David, 22130 Corseul (FR)
(74) Mandataire: CAPRI
(86) Numéro de dépôt international: PCT/FR2019/052837
(87) Numéro de publication internationale: WO 2020/109737

(56) Documents cités:
- WO-A1-02/24265
- WO-A1-92/09323
- WO-A1-2016/030521
- WO-A1-2017/178764
- US-A1- 2018 228 989

## Description

La présente invention concerne un dispositif de distribution de produit fluide synchronisé avec l'inhalation, et plus particulièrement un dispositif d'inhalation du type aérosol synchronisé avec l'inhalation.

Les dispositifs actionnés par l'inhalation, désignés généralement par le terme BAI (signifiant "Breath Actuated Inhaler"), sont bien connus dans l'état de la technique. L'avantage principal de ce type de dispositif est que la distribution du produit est synchronisée avec l'inhalation du patient, pour garantir une bonne distribution du produit dans les voies respiratoires. Ainsi, dans le domaine des dispositifs aérosol, c'est-à-dire ceux dans lesquels le produit est distribué à l'aide d'un gaz propulseur, ainsi que dans le domaine des inhalateurs de poudre sèche, dans lesquels le produit est distribué au moyen du flux d'inhalation généré par l'utilisateur, de nombreux types de dispositifs de déclenchement par l'inhalation ont été proposés. Ces dispositifs présentent toutefois l'inconvénient de présenter des risques de contamination, par exemple bactérienne et/ou microbienne, au niveau du système BAI. Une solution est de laver ledit système BAI après chaque utilisation, mais de nombreux dispositifs ne sont pas adaptés à cette solution, et de l'eau stagnante dans le dispositif est également source de contamination potentielle.

Les documents WO2017178764, WO2016030521, WO0224265, US2018228989 et WO9209323 décrivent des dispositifs de l'état de la technique.

La présente invention a pour but de fournir un dispositif de distribution de produit fluide synchronisé avec l'inhalation qui ne reproduit pas les inconvénients susmentionnés.

La présente invention a aussi pour but de fournir un dispositif de distribution de produit fluide synchronisé avec l'inhalation qui améliore la fiabilité de fonctionnement, en garantissant un actionnement efficace à chaque inhalation.

La présente invention a aussi pour but de fournir un dispositif de distribution de produit fluide synchronisé avec l'inhalation qui minimise les risques de contamination, par exemple bactérienne et/ou microbienne, du produit distribué à chaque actionnement.

La présente invention a également pour but de fournir un dispositif de distribution de produit fluide synchronisé avec l'inhalation qui peut être lavé après chaque utilisation.

La présente invention a également pour but de fournir un dispositif de distribution de produit fluide synchronisé avec l'inhalation qui soit simple et peu coûteux à fabriquer et à assembler.

La présente a donc pour objet un dispositif de distribution de produit fluide synchronisé avec l'inhalation, comportant un corps pourvu d'un embout buccal, au moins un réservoir de produit contenant du produit fluide, et des moyens de distribution d'une dose de produit fluide à chaque actionnement, ledit dispositif comprenant des moyens de blocage desdits moyens de distribution, ledit dispositif comprenant un système de déclenchement commandé par l'inhalation comportant un organe sensible à l'inhalation, déformable et/ou déplaçable sous l'effet de l'inhalation, ledit organe sensible à l'inhalation coopérant avec lesdits moyens de blocage de sorte que lorsque ledit organe sensible à l'inhalation se déforme et/ou se déplace sous l'effet de l'inhalation, il permet l'actionnement desdits moyens de distribution, ledit dispositif comportant un élément de protection dudit système de déclenchement commandé par l'inhalation, ledit élément de protection étant disposé entre ledit embout buccal et ledit organe sensible à l'inhalation, ledit élément de protection étant réalisé en un matériau poreux laissant passer l'air mais bloquant le passage d'eau, de poussières et de corps étrangers.

Avantageusement, ledit élément de protection est réalisé en un matériau fritté à pores ouverts.

Avantageusement, ledit matériau fritté à pores ouverts comporte un matériau du type polyoléfines, tel que le polyéthylène (PE), le polypropylène (PP) ou un mélange des deux, et/ou du type polytétrafluoroéthylène (PTFE).

Avantageusement, ledit élément de protection est soumis à un traitement antibactérien.

Avantageusement, ledit réservoir de produit, contenant un produit fluide et un gaz propulseur, est monté axialement coulissant par rapport audit corps, une valve doseuse, comportant une soupape, étant assemblée sur ledit réservoir pour distribuer sélectivement le produit fluide.

Avantageusement, le dispositif comporte un élément de blocage déplaçable et/ou déformable entre une position de blocage, dans laquelle ladite valve doseuse ne peut pas être actionnée, et une position d'actionnement, dans laquelle ladite valve doseuse peut être actionnée, un élément déclencheur déplaçable et/ou déformable entre une position de verrouillage, dans laquelle il bloque ledit élément de blocage dans sa position de blocage, et une position de libération, dans laquelle il ne bloque pas ledit élément de blocage, et ledit organe sensible à l'inhalation coopérant avec ledit élément déclencheur, de sorte que lorsque ledit organe sensible à l'inhalation se déforme et/ou se déplace, il déplace et/ou déforme ledit élément déclencheur vers sa position de libération, permettant ainsi le déplacement et/ou la déformation dudit élément de blocage de sa position de blocage vers sa position d'actionnement.

Avantageusement, un organe d'actionnement est monté axialement déplaçable, notamment coulissant, dans ledit corps entre une position de repos et une position chargée, un ressort étant disposé entre ledit organe d'actionnement et ledit réservoir ou un élément solidaire dudit réservoir, de sorte que lorsque ledit organe d'actionnement se déplace vers sa position chargée, ledit ressort est comprimé, pour transmettre une force axiale audit réservoir.

Avantageusement, une poignée d'actionnement latérale est montée déplaçable en rotation et/ou en translation sur ledit corps entre une position de repos et une position d'utilisation, un déplacement de ladite poignée d'actionnement latérale vers sa position d'utilisation déplaçant axialement ledit organe d'actionnement vers sa position chargée.

Avantageusement, ledit organe sensible à l'inhalation comporte une membrane déformable définissant une chambre d'air déformable, ladite membrane déformable étant fixée audit élément déclencheur, ladite membrane déformable étant déformée lors de l'inhalation.

Ces caractéristiques et avantages et d'autres apparaîtront plus clairement au cours de la description détaillée suivante, faite en référence aux dessins joints, donnés à titre d'exemples non limitatifs, et sur lesquels :
la Figure 1 est une vue schématique en section d'un dispositif de distribution de produit fluide, en position de repos, selon un mode de réalisation avantageux,
la Figure 2 est une vue schématique partielle en perspective éclatée d'une partie du dispositif de la figure 1,
la Figure 3 est une vue schématique partielle en perspective découpée d'une partie du dispositif de la figure 1,
la Figure 4 est une vue très schématique d'un dispositif de distribution de produit fluide, selon un mode de réalisation avantageux,
la Figure 5 est une vue similaire à celle de la figure 4, montrant un autre mode de réalisation avantageux, et
la Figure 6 est une vue similaire à celles des figures 4 et 5, montrant encore un autre mode de réalisation avantageux.

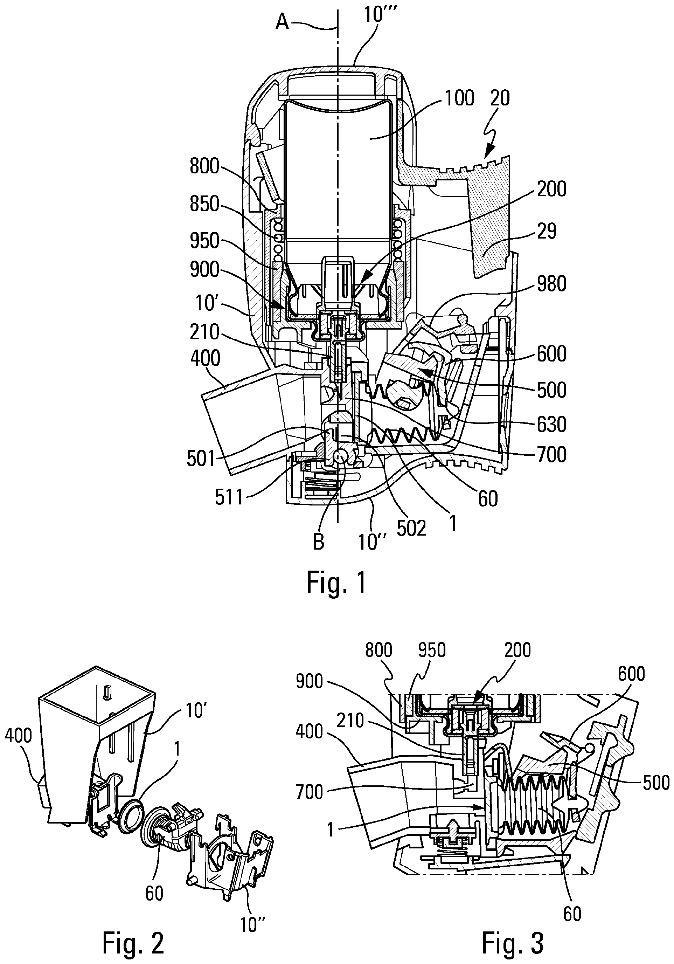

Dans la description, les termes "supérieur" et "inférieur" se réfèrent à la position droite du dispositif représentée sur la figure 1. Les termes "axial" et "radial", sauf si autrement spécifié, se réfèrent à l'axe central vertical A de la valve représenté sur la figure 1. Les termes "proximal" et "distal" se réfèrent à l'embout buccal.

Les figures représentent des modes de réalisation avantageux de l'invention, mais il est entendu que l'une ou plusieurs des pièces constitutives décrites ci-après pourrai(en)t être réalisée(s) différemment tout en procurant des fonctions similaires voire identiques.

Seul le mode de réalisation des figures 1 à 3 est représenté et décrit ci-après en détails, mais il est entendu que la présente invention n'est pas limitée à ce mode de réalisation particulier, et que tout type de dispositif de distribution, avec ou sans gaz propulseur, et tout type de système BAI sont potentiellement adaptables à la présente invention.

De manière connue, un dispositif de distribution de produit fluide synchronisé avec l'inhalation comporte un corps 10 pourvu d'un embout buccal 400, au moins un réservoir de produit 100 contenant du produit fluide, et des moyens de distribution 200 d'une dose de produit fluide à chaque actionnement. Le dispositif comprend des moyens de blocage 500, 600 de ces moyens de distribution 200, et un système de déclenchement commandé par l'inhalation. Celui-ci comporte un organe sensible à l'inhalation 60, déformable et/ou déplaçable sous l'effet de l'inhalation, qui coopère avec les moyens de blocage de telle sorte que lorsque ledit organe sensible à l'inhalation 60 se déforme et/ou se déplace sous l'effet de l'inhalation, il permet l'actionnement desdits moyens de distribution 200.

Selon l'invention, le système de déclenchement commandé par l'inhalation comporte un élément de protection 1 disposé entre l'embout buccal 400 et l'organe sensible à l'inhalation 60, ledit élément de protection 1 étant réalisé en un matériau poreux laissant passer l'air mais bloquant le passage d'eau, de poussières et de corps étrangers.

Avantageusement, l'élément de protection 1 est une membrane réalisée en un matériau fritté à pores ouverts. Ce type de matériaux permet de perturber le moins possible le flux d'air d'inhalation et limite ainsi la hausse de résistance du système, tout en rendant le passage d'eau impossible.

Ce matériau peut comporter un matériau du type polyoléfines, tel que le polyéthylène (PE), le polypropylène (PP) ou un mélange des deux, et/ou du type polytétrafluoroéthylène (PTFE). Ces matières ont un comportement naturellement hydrophobe et captent peu ou pas du tout l'humidité, ce qui va dans le sens d'éviter toute stagnation de liquide pour éviter les développements bactériens et/ou microbiens.

Des matériaux possibles comprennent un Porex^{®}, ou le Tyvek^{®} de chez Dupont de Nemours. Plus généralement, on peut envisager tout matériau réalisé à partir de fibre de polyéthylène haute densité (PEHD) non tissé. Là aussi ce type de matière permet le passage de l'air avec un débit maitrisé en offrant la protection attendue. D'autres matériaux comprennent le Sabeu Trakecht^{®}, qui forme des filtres micro perforés en polymères à la porosité contrôlée, ou le Millipore^{®}, série AP, filtre à base de borosilicate et liant acrylique.

Avantageusement, l'élément de protection 1 est soumis à un traitement antibactérien.

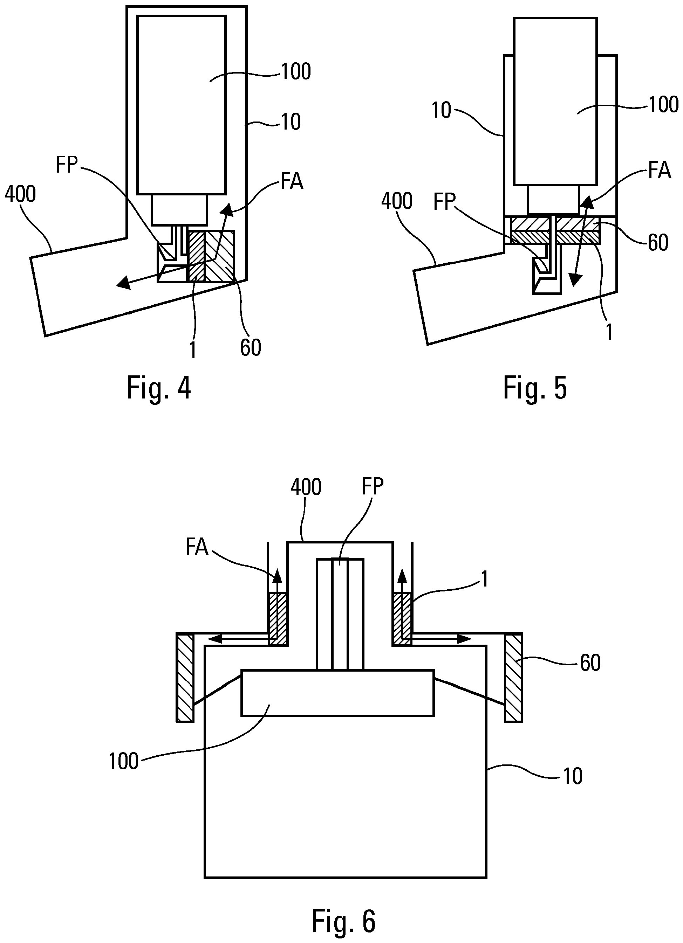

Les figures 4 à 6 montrent de manière très schématique divers dispositifs auxquels la présente invention peut s'appliquer.

Ainsi, la figure 4 illustre un dispositif tel que décrit ci-après en référence aux figures 1 à 3, dans lequel l'élément de protection poreux 1 est en communication fluidique avec le flux d'air FA et le flux de produit FP.

La figure 5 illustre un dispositif dans lequel l'élément de protection poreux 1 est en communication fluidique avec le flux d'air FA mais pas avec le flux de produit FP.

Enfin, la figure 6 illustre un dispositif du type inhalateur de poudre sèche, dans lequel l'élément de protection poreux 1 est en communication fluidique avec le flux d'air FA mais pas avec le flux de produit FP.

Dans tous les cas, l'élément de protection poreux 1 protège l'organe sensible à l'inhalation 60. Cette protection permet, entre autres bénéfices, de conserver propres tous les éléments disposés derrière cet élément de protection poreux 1. Ceci permet notamment le nettoyage et évite la prolifération bactérienne et/ou microbienne.

Un exemple de réalisation avantageux sera maintenant décrit en détails, en référence aux figures 1 à 3.

Dans cet exemple, le dispositif comporte un corps 10 pourvu d'un embout buccal 400.

Le corps 10 peut être réalisé en une seule pièce ou en plusieurs pièces assemblées les unes aux autres. Dans l'exemple représenté, non limitatif, le corps 10 comporte trois parties, une partie centrale 10', une partie inférieure 10" et une partie supérieure 10‴. Dans la suite de la description, le corps sera désigné de manière globale par la référence numérique 10.

L'embout buccal 400 définit un orifice de distribution à travers lequel l'utilisateur va inhaler lors de l'utilisation du dispositif. L'embout buccal 400 peut être réalisé d'une pièce avec le corps 10. Dans les exemples représentés sur les dessins, il est formé sur la partie inférieure de corps 10". Un capot de protection amovible (non représenté) peut être prévu sur ledit embout buccal 400, notamment lors des périodes de stockage, que l'utilisateur va retirer avant utilisation.

Le corps 10 contient un réservoir 100, contenant le produit à distribuer et un gaz propulseur, tel qu'un gaz du type HFA, une valve doseuse 200 étant montée sur ledit réservoir 100 pour distribuer sélectivement le produit. La valve doseuse 200 comporte un corps de valve et une soupape 210 axialement déplaçable lors de l'actionnement par rapport audit corps de valve, et donc par rapport audit réservoir 100. Cette valve doseuse 200 peut être d'un type quelconque approprié. Elle est fixée au réservoir 100 par un élément de fixation, de préférence une capsule sertie, de préférence avec interposition d'un joint de col.

Avantageusement, lors de l'actionnement, la soupape 210 est fixe par rapport au corps 10, et c'est le réservoir 100 qui est déplacé axialement par rapport au corps 10 entre une position distale, qui est la position de repos, et une position proximale, qui est la position d'actionnement.

L'orifice de sortie de la soupape 210 de ladite valve doseuse 200 est relié via un canal audit embout buccal 400, à travers lequel l'utilisateur inhale le produit distribué. De manière connue, ladite soupape 210 est reçue dans un puits de soupape 700 qui définit au moins partiellement ledit canal.

Le dispositif comporte une bague 900 qui est avantageusement fixée autour dudit élément de fixation, par exemple par encliquetage au moyen de pattes d'encliquetage. Avantageusement, une frette 950 est emmanchée autour de ladite bague 900, pour maintenir lesdites pattes d'encliquetage en position encliquetée.

Un organe d'actionnement 800 est avantageusement assemblé autour du réservoir 100. Cet organe d'actionnement 800 est disposé dans le corps 10 autour du réservoir 100, avec un ressort 850 disposé entre son fond et le réservoir 100 ou un élément solidaire dudit réservoir 100, tel que la bague 900 ou la frette 950. L'organe d'actionnement 800 est déplaçable axialement, notamment coulissant, par rapport audit réservoir 100 entre une position de repos et une position chargée. Ainsi, lorsque l'utilisateur souhaite actionner la valve doseuse 200, il appuie sur ledit organe d'actionnement 800. Ceci va le déplacer axialement vers sa position chargée et ainsi comprimer ledit ressort 850, qui va donc transmettre une force axiale F audit réservoir 100, notamment via ladite frette 950 dans l'exemple représenté. Cette force axiale F est sensiblement la même à chaque actionnement. Tant que l'utilisateur maintient son appui sur ledit organe d'actionnement 800, ledit ressort 850 est comprimé et sollicite axialement ledit réservoir 100 vers sa position d'actionnement.

Une poignée d'actionnement latérale 20 est avantageusement montée déplaçable en rotation et/ou en translation sur le corps 10. Cette poignée 20, lorsque déplacée de sa position de repos, visible notamment sur la figure 1, vers sa position d'utilisation, déplace axialement ledit organe d'actionnement 800 pour comprimer le ressort 850.

Avantageusement, la poignée 20 est déplaçable à la fois en rotation et en translation. Ceci permet de démultiplier l'effort de l'utilisateur, tout en gardant un encombrement réduit. Cette démultiplication permet d'actionner la valve 200 avec un effort bien inférieur à celui qui serait nécessaire si l'on devait appuyer axialement sur le fond du réservoir 100. En particulier, dans l'exemple représenté, l'effort nécessaire pour actionner axialement la valve 200 est typiquement de 40-45N (lié à la raideur du ressort 850) alors que l'effort pour actionner la poignée d'actionnement latérale 20 est seulement d'environ 15N. La démultiplication est donc ici d'environ un facteur trois. Il est possible d'augmenter encore ce ratio, notamment en jouant sur la géométrie des différentes pièces.

Tant que l'utilisateur maintient son appui sur ladite poignée, ledit ressort 850 est comprimé et sollicite axialement ledit réservoir 100 vers sa position d'actionnement.

Après chaque actionnement, lorsque l'utilisateur relâche sa pression sur la poignée 20, ce qui se fait naturellement, celle-ci revient automatiquement vers sa position de repos sous l'effet du ressort 850. Ceci permet d'éviter le risque qu'après actionnement de la valve doseuse 200, cette dernière reste en position actionnée, ce qui pourrait avoir pour conséquence que la chambre de la valve se remplit d'air et que la dose suivante est incomplète voire que la valve fuit. Ceci est une des problématiques rencontrées actuellement par les dispositifs existants sur le marché.

Le dispositif comporte un élément de blocage 500 déplaçable et/ou déformable entre une position de blocage, dans laquelle ladite valve doseuse 200 ne peut pas être actionnée, et une position d'actionnement, dans laquelle ladite valve doseuse 200 peut être actionnée. En position de repos, ledit élément de blocage 500 est en position de blocage, et c'est l'inhalation de l'utilisateur à travers l'embout buccal 400 qui déplace et/ou déforme ledit élément de blocage 500 vers sa position d'actionnement. En d'autres termes, tant que l'utilisateur n'a pas inhalé, l'actionnement de la valve doseuse 200 est impossible, et ce n'est que lorsqu'il inhale que ladite valve doseuse 200 peut être actionnée, par déplacement axial du réservoir 100 dans le corps 10.

Comme cela sera décrit plus en détails ci-après, l'élément de blocage 500, en position de blocage, empêche le déplacement axial du réservoir 100 dans le corps 10. Lors de l'inhalation, cet élément de blocage 500 est déplacé et/ou déformé de telle sorte qu'il ne bloque plus le déplacement axial du réservoir 100 dans le corps 10. Ainsi, après inhalation, un tel déplacement axial du réservoir 100 provoque l'actionnement de la valve doseuse 200 et la distribution d'une dose de produit synchronisée avec cette inhalation.

Ainsi, en l'absence d'inhalation, il n'y a aucun risque qu'une dose de produit actif soit perdue par un actionnement malencontreux ou incomplet dans lequel l'utilisateur n'exercerait pas d'inhalation. L'actionnement de la valve 200 et l'expulsion d'une dose de produit fluide ne sont donc possibles que si l'utilisateur inhale et que simultanément il actionne la poignée d'actionnement 20. En variante, on pourrait aussi envisager que l'utilisateur appuie axialement directement sur le fond du réservoir, ou alors utiliser un système d'actionnement automatique, qui appliquerait un appui axial sur le réservoir indépendamment de l'utilisateur.

Le dispositif comporte un système de déclenchement commandé par l'inhalation de l'utilisateur, qui est destiné à déplacer et/ou déformer ledit élément de blocage 500 de sa position de blocage vers sa position d'actionnement, lorsque l'utilisateur inhale à travers l'embout buccal 400.

Ce système de déclenchement comporte un organe sensible à l'inhalation 60 déformable et/ou déplaçable sous l'effet de l'inhalation, cet organe sensible à l'inhalation 60 étant adapté, lorsqu'il se déforme et/ou se déplace, à permettre le déplacement et/ou la déformation dudit élément de blocage 500 de sa position de blocage vers sa position d'actionnement.

Selon l'invention, ledit organe sensible à l'inhalation 60 est pourvu, du côté relié à l'embout buccal 400, d'un élément de protection poreux 1, avantageusement réalisé sous la forme d'une membrane.

Comme cela sera décrit plus en détails ci-après, l'organe sensible à l'inhalation peut être réalisé sous la forme d'une chambre d'air déformable 60, par exemple un soufflet ou une poche déformable.

De cette manière, le système de déclenchement commandé par l'inhalation n'est pas situé dans le flux de l'aspiration de l'utilisateur mais est formé par une chambre spécifique, à savoir la chambre d'air 60. Ceci diffère des systèmes fonctionnant à l'aide d'un volet qui se déplace/déforme dans le flux d'aspiration, dans lesquels, après déclenchement, l'utilisateur va aspirer l'air qui se trouve de chaque côté du volet. Ici, le système fonctionne en dépression et l'utilisateur n'aspire que le petit volume d'air qui était à l'intérieur de la chambre d'air 60 avant sa déformation. Le système selon l'invention est ainsi beaucoup plus stable et performant.

L'élément de blocage 500 est avantageusement monté pivotant autour d'un axe B sur le corps 10 entre une position de blocage et une position d'actionnement. Dans l'exemple représenté, ledit axe B peut être formé par des projections prévues sur une surface inférieure du corps 10, l'élément de blocage 500 comportant des profils complémentaires 511 adaptés à pivoter sur lesdites projections. D'autres mises en oeuvre sont aussi possibles.

L'élément de blocage 500 comporte au moins une, de préférence deux extensions de blocage 501, qui coopère chacune en position de blocage avec une projection axiale de ladite bague 900 solidaire du réservoir 100.

Lorsque l'élément de blocage 500 se déplace vers sa position d'actionnement, notamment par pivotement autour de l'axe B, chaque extension de blocage 501 se déplace hors de contact avec la projection axiale respective de ladite bague 900. En particulier, ledit élément de blocage 500 comporte de manière adjacente à chaque extension de blocage 501 un évidement axial 502 dans lequel la projection axiale respective de ladite bague 900 peut coulisser axialement, pour ainsi permettre un coulissement axial dudit réservoir 100 dans ledit corps 10, provoquant l'actionnement de la valve 200 et la distribution d'une dose de produit fluide.

L'élément de blocage 500 est retenu en position de blocage par un élément déclencheur 600. Cet élément déclencheur 600 est avantageusement monté pivotant autour d'un axe sur le corps 10, entre une position de verrouillage, dans laquelle il bloque ledit élément de blocage 500 dans sa position de blocage, et une position de libération, dans laquelle il ne bloque plus ledit élément de blocage 500.

Avantageusement, les axes de l'élément de blocage 500 et de l'élément déclencheur 600 sont parallèles.

Le système de blocage de la présente invention comporte donc deux étages : un premier étage formé par la serrure entre l'élément de blocage 500 et l'élément déclencheur 600, et un second étage formé par le blocage entre l'élément de blocage 500 et le réservoir 100, via la bague 900.

Ce système de blocage permet le déverrouillage d'un gros effort (typiquement environ 40-45N) par un petit effort généré par l'inhalation. L'élément de blocage 500 arrête la translation du réservoir 100 lorsqu'il est soumis à un effort F (de 45N par exemple) par l'appui par l'utilisateur sur l'organe d'actionnement 800, de préférence via la poignée d'actionnement 20. Cet élément de blocage 500 interagit avec l'élément déclencheur 600 et est bloqué/libéré par celui-ci. Le déplacement dudit élément déclencheur 600 est commandé par l'inhalation.

La géométrie du système de blocage permet une amplification (effort déverrouillé/effort déverrouillant) très importante, typiquement de l'ordre de 100.

La force de déverrouillage générée par l'inhalation est appliquée sur l'élément déclencheur 600 par la membrane déformable 60, de préférence en un point 630 éloigné de l'axe de pivotement de l'élément déclencheur 600.

Grâce à ce système d'effort de la serrure, l'effort nécessaire pour faire pivoter l'élément déclencheur 600 est très faible et peut être généré par la membrane déformable 60, qui permet de transformer la dépression générée par l'inhalation en force de déverrouillage.

Dans le mode de réalisation représenté sur la figure 1, l'organe sensible à l'inhalation 60 est réalisé sous la forme d'une chambre d'air déformable. Avantageusement, cette chambre d'air comprend une membrane déformable qui est reliée d'une part à ladite partie inférieure de corps 10' et d'autre part audit élément déclencheur 600. Avantageusement, l'organe sensible à l'inhalation 60 a une forme de soufflet et forme une chambre sensiblement étanche. D'autres formes sont possibles, notamment une simple poche ou diaphragme. Un plot peut fixer l'organe sensible à l'inhalation 60 à un orifice ou bord 630 dudit élément déclencheur 600.

Lors de l'inhalation, l'organe sensible à l'inhalation 60 se déforme et/ou se contracte sous l'effet de la dépression générée par l'inhalation, provoquant le déplacement de l'élément déclencheur 600 de sa position de verrouillage vers sa position de libération. Ceci permet d'ouvrir la serrure définie entre l'élément de blocage 500 et l'élément déclencheur 600, et donc le déplacement dudit élément de blocage 500 de sa position de blocage vers sa position d'actionnement.

La valve 200 n'est donc actionnée qu'au moment de l'inhalation, de sorte que la dose de produit fluide est expulsée hors de l'orifice de distribution simultanément à l'inhalation.

Avantageusement, le dispositif comporte un organe bloquant 980 déplaçable et/ou déformable entre une position bloquante et une position non bloquante. En position bloquante, cet organe bloquant 980 coopère avec l'élément déclencheur 600 pour l'empêcher de se déplacer vers sa position de libération. La poignée d'actionnement latérale 20 comporte avantageusement une projection 29 coopérant avec ledit organe bloquant 980 lorsque ladite poignée d'actionnement latérale 20 est déplacée vers sa position d'utilisation. Ceci déplace et/ou déforme ledit organe bloquant 980 vers sa position non bloquante.

Ainsi, si l'utilisateur inhale sans avoir exercé l'appui axial sur le réservoir 100, la serrure ne sera pas débloquée, puisque l'élément déclencheur 600 ne peut pas pivoter. La chambre d'air 60 étant sensiblement étanche, l'utilisateur se rend compte très vite qu'il ne peut inhaler correctement à travers l'embout buccal 400, ce qui lui rappelle qu'il faut d'abord actionner la poignée 20 avant d'inhaler. Lorsque l'utilisateur appuie sur la poignée 20, il déplace l'organe bloquant 980 dans sa position non bloquante. Une inhalation provoquera alors un pivotement de l'élément déclencheur 600, et donc l'actionnement du dispositif, comme expliqué précédemment.

Avantageusement, l'organe bloquant 980 comporte un élément élastique (non représenté), tel qu'un ressort de torsion, qui sollicite élastiquement ledit organe bloquant 980 vers sa position bloquante, de sorte que lorsque l'utilisateur relâche la poignée d'actionnement 20, ledit organe bloquant 980 revient automatiquement dans sa position bloquante. Avantageusement, ceci ramène également l'élément déclencheur 600 dans sa position de verrouillage, par exemple via une lame flexible appropriée.

Lorsque l'utilisateur souhaite utiliser le dispositif, il place l'embout buccal 400 dans sa bouche, et exerce manuellement un appui axial sur la poignée d'actionnement 20. Le réservoir 100 est bloqué et empêché de coulisser axialement dans le corps 10 par les extensions de blocage 501 de l'élément de blocage 500 qui bloquent axialement les projections axiales de la bague 900. Parallèlement, le blocage de l'élément déclencheur 600 est supprimé par le déplacement de l'organe bloquant 980.

Lorsque l'utilisateur inhale à travers l'embout buccal 400, il va déformer l'organe sensible à l'inhalation 60, ce qui va faire pivoter l'élément déclencheur 600 fixé à ladite membrane déformable 60. Ce déplacement de l'élément déclencheur 600 va libérer la serrure formée entre l'élément déclencheur 600 et l'élément de blocage 500. Sous l'effet de la force axiale F transmise par le réservoir 100, l'élément de blocage 500 va pivoter permettant le coulissement axial du réservoir 100 dans le corps 10 vers sa position de distribution, et donc l'actionnement de la valve 200.

En fin d'inhalation, lorsque l'utilisateur relâche sa pression sur le fond du réservoir 100, notamment en relâchant sa pression sur la poignée 20, ledit réservoir 100 remonte axialement dans le corps 10 en direction de sa position de repos sous l'effet du ressort de rappel de la valve 200, et simultanément la soupape 210 de la valve doseuse revient en position de repos, en chargeant une nouvelle dose de produit fluide dans la chambre de valve. L'élément déclencheur 600 est ramené dans sa position initiale, notamment par l'élasticité de la membrane 60 et/ou la lame élastique de l'organe bloquant 980. L'élément de blocage 500 revient dans sa position de blocage, via une extension de réarmement de l'organe bloquant 980.

Le dispositif est alors prêt pour une autre utilisation.

Il est à noter que le dispositif pourrait comporter un compteur de doses électronique, avantageusement assemblé dans le corps ou dans la poignée. Ce compteur peut notamment détecter les déplacements du réservoir. En variante, le compteur pourrait être relié à un capteur, notamment un capteur à membrane, qui détecte la distribution de la dose de produit fluide, par exemple dans le puits de soupape. D'autres moyens d'actionner un tel compteur électronique sont aussi possibles, par exemple la détection du déplacement de la soupape de la valve doseuse par rapport au corps de valve.

La présente invention est notamment applicable pour le traitement des crises d'asthme ou de BPCO (Broncho Pneumopathie Obstructive) en utilisation avec des formulations de type salbutamol, aclidinium, formoterol, tiotropium, budesonide, fluticasone, indacaterol, glycopyrronium, salmeterol, umeclidinium bromide, vilanterol, olodaterol, striverdi, ou toute combinaison de ces formulations.

La présente invention a été décrite en référence à des modes de réalisation et variantes avantageux, mais il est entendu qu'un homme du métier peut y apporter toutes modifications, sans sortir du cadre de la présente invention tel que défini par les revendications annexées.

## Revendications

1. Dispositif de distribution de produit fluide synchronisé avec l'inhalation, comportant un corps (10) pourvu d'un embout buccal (400), au moins un réservoir de produit (100) contenant du produit fluide, et des moyens de distribution (200) d'une dose de produit fluide à chaque actionnement, ledit dispositif comprenant des moyens de blocage (500 ; 600) desdits moyens de distribution (200), ledit dispositif comprenant un système de déclenchement commandé par l'inhalation comportant un organe sensible à l'inhalation (60), déformable et/ou déplaçable sous l'effet de l'inhalation, ledit organe sensible à l'inhalation (60) coopérant avec lesdits moyens de blocage (500 ; 600) de sorte que lorsque ledit organe sensible à l'inhalation (60) se déforme et/ou se déplace sous l'effet de l'inhalation, il permet l'actionnement desdits moyens de distribution (200), **caractérisé en ce que** ledit dispositif comporte un élément de protection (1) dudit système de déclenchement commandé par l'inhalation, ledit élément de protection (1) étant disposé entre ledit embout buccal (400) et ledit organe sensible à l'inhalation (60), ledit élément de protection (1) étant réalisé en un matériau poreux laissant passer l'air mais bloquant le passage d'eau, de poussières et de corps étrangers.

2. Dispositif selon la revendication 1, dans lequel ledit élément de protection (1) est réalisé en un matériau fritté à pores ouverts.

3. Dispositif selon la revendication 2, dans lequel ledit matériau fritté à pores ouverts comporte un matériau du type polyoléfines, tel que le polyéthylène (PE), le polypropylène (PP) ou un mélange des deux, et/ou du type polytétrafluoroéthylène (PTFE).

4. Dispositif selon l'une quelconque des revendications précédentes, dans lequel ledit élément de protection (1) est soumis à un traitement antibactérien.

5. Dispositif selon l'une quelconque des revendications précédentes, dans lequel ledit réservoir de produit (100), contenant un produit fluide et un gaz propulseur, est monté axialement coulissant par rapport audit corps (10), une valve doseuse (200), comportant une soupape (210), étant assemblée sur ledit réservoir (100) pour distribuer sélectivement le produit fluide.

6. Dispositif selon la revendication 5, comportant un élément de blocage (500) déplaçable et/ou déformable entre une position de blocage, dans laquelle ladite valve doseuse (200) ne peut pas être actionnée, et une position d'actionnement, dans laquelle ladite valve doseuse (200) peut être actionnée, un élément déclencheur (600) déplaçable et/ou déformable entre une position de verrouillage, dans laquelle il bloque ledit élément de blocage (500) dans sa position de blocage, et une position de libération, dans laquelle il ne bloque pas ledit élément de blocage (500), et ledit organe sensible à l'inhalation (60) coopérant avec ledit élément déclencheur (600), de sorte que lorsque ledit organe sensible à l'inhalation (60) se déforme et/ou se déplace, il déplace et/ou déforme ledit élément déclencheur (600) vers sa position de libération, permettant ainsi le déplacement et/ou la déformation dudit élément de blocage (500) de sa position de blocage vers sa position d'actionnement.

7. Dispositif selon l'une quelconque des revendications précédentes, dans lequel un organe d'actionnement (800) est monté axialement déplaçable, notamment coulissant, dans ledit corps (10) entre une position de repos et une position chargée, un ressort (850) étant disposé entre ledit organe d'actionnement (800) et ledit réservoir (100) ou un élément (900 ; 950) solidaire dudit réservoir (100), de sorte que lorsque ledit organe d'actionnement (800) se déplace vers sa position chargée, ledit ressort (850) est comprimé, pour transmettre une force axiale (F) audit réservoir (100).

8. Dispositif selon la revendication 7, dans lequel une poignée d'actionnement latérale (20) est montée déplaçable en rotation et/ou en translation sur ledit corps (10) entre une position de repos et une position d'utilisation, un déplacement de ladite poignée d'actionnement latérale (20) vers sa position d'utilisation déplaçant axialement ledit organe d'actionnement (800) vers sa position chargée.

9. Dispositif selon l'une quelconque des revendications précédentes, dans lequel ledit organe sensible à l'inhalation (60) comporte une membrane déformable définissant une chambre d'air déformable, ladite membrane déformable étant fixée audit élément déclencheur (600), ladite membrane déformable étant déformée lors de l'inhalation.

## Patentansprüche

1. Vorrichtung zur inhalationssynchronisierten Ausgabe von Fluidprodukt, aufweisend einen Körper (10), der mit einem Mundstück (400) versehen ist, wenigstens einen Produktbehälter (100), der Fluidprodukt enthält, und Mittel zur Ausgabe (200) einer Dosis Fluidprodukt bei jeder Betätigung, wobei die Vorrichtung Mittel zum Blockieren (500; 600) der Ausgabemittel (200) umfasst, wobei die Vorrichtung ein inhalationsgesteuertes Auslösesystem umfasst, das ein inhalationsempfindliches Organ (60) aufweist, das unter der Wirkung der Inhalation verformbar und/oder bewegbar ist, wobei das inhalationsempfindliche Organ (60) mit den Blockiermitteln (500; 600) so zusammenwirkt, dass es, wenn sich das inhalationsempfindliche Organ (60) unter der Wirkung der Inhalation verformt und/oder bewegt, die Betätigung der Ausgabemittel (200) ermöglicht, **dadurch gekennzeichnet, dass** die Vorrichtung ein Element zum Schutz (1) des inhalationsgesteuerten Auslösesystems aufweist, wobei das Schutzelement (1) zwischen dem Mundstück (400) und dem inhalationsempfindlichen Organ (60) angeordnet ist, wobei das Schutzelement (1) aus einem porösen Material hergestellt ist, das Luft durchlässt, jedoch den Durchtritt von Wasser, Staub und Fremdkörpern blockiert.

2. Vorrichtung nach Anspruch 1, wobei das Schutzelement (1) aus einem offenporigen Sintermaterial hergestellt ist.

3. Vorrichtung nach Anspruch 2, wobei das offenporige Sintermaterial ein Polyolefinmaterial wie etwa Polyethylen (PE), Polypropylen (PP) oder ein Gemisch von beiden, und/oder Polytetrafluorethylen (PTFE) aufweist.

4. Vorrichtung nach einem der vorhergehenden Ansprüche, wobei das Schutzelement (1) antibakteriell behandelt ist.

5. Vorrichtung nach einem der vorhergehenden Ansprüche, wobei der Produktbehälter (100), der ein Fluidprodukt und ein Treibgas enthält, bezogen auf den Körper (10) axial gleitend gelagert ist, wobei ein Dosierventil (200) mit einer Ventileinrichtung (210) am Behälter (100) angebracht ist, um das Fluidprodukt selektiv auszugeben.

6. Vorrichtung nach Anspruch 5, aufweisend ein Blockierelement (500), das zwischen einer Blockageposition, in der das Dosierventil (200) nicht betätigt werden kann, und einer Betätigungsposition, in der das Dosierventil (200) betätigt werden kann, bewegbar und/oder verformbar ist, ein Auslöseelement (600), das zwischen einer Verriegelungsposition, in der es das Blockierelement (500) in dessen Blockageposition blockiert, und einer Freigabeposition, in der es das Blockierelement (500) nicht blockiert, bewegbar und/oder verformbar ist, und das inhalationsempfindliche Organ (60), das mit dem Auslöseelement (600) so zusammenwirkt, dass es, wenn sich das inhalationsempfindliche Organ (60) verformt und/oder bewegt, das Auslöseelement (600) zu dessen Freigabeposition bewegt und/oder verformt, wodurch die Bewegung und/oder Verformung des Blockierelements (500) aus dessen Blockageposition zu dessen Betätigungsposition ermöglicht wird.

7. Vorrichtung nach einem der vorhergehenden Ansprüche, wobei ein Betätigungsorgan (800) axial bewegbar, insbesondere gleitend, im Körper (10) zwischen einer Ruheposition und einer belasteten Position gelagert ist, wobei eine Feder (850) zwischen dem Betätigungsorgan (800) und dem Behälter (100) oder einem mit dem Behälter (100) fest verbundenen Element (900; 950) so angeordnet ist, dass, wenn sich das Betätigungsorgan (800) zu seiner belasteten Position bewegt, die Feder (850) zusammengedrückt wird, um eine axiale Kraft (F) auf den Behälter (100) zu übertragen.

8. Vorrichtung nach Anspruch 7, wobei ein seitlicher Betätigungsgriff (20) dreh- und/oder verschiebbar am Körper (10) zwischen einer Ruheposition und einer Gebrauchsposition gelagert ist, wobei eine Bewegung des seitlichen Betätigungsgriffs (20) zu dessen Gebrauchsposition das Betätigungsorgan (800) axial zu dessen belasteten Position bewegt.

9. Vorrichtung nach einem der vorhergehenden Ansprüche, wobei das inhalationsempfindliche Organ (60) eine verformbare Membran aufweist, die eine verformbare Luftkammer definiert, wobei die verformbare Membran am Auslöseelement (600) befestigt ist, wobei die verformbare Membran bei der Inhalation verformt wird.

## Claims

1. Inhalation-synchronized fluid product dispenser device comprising a body (10) provided with a mouthpiece (400), at least one fluid reservoir (100) containing fluid, and dispenser means (200) for dispensing a dose of fluid on each actuation, said device comprising blocking means (500; 600) for said dispenser means (200), SAID device comprising an inhalation-controlled trigger system comprising an inhalation-sensitive member (60) that is deformable and/or movable under the effect of inhaling, said inhalation-sensitive member co-operating with said blocking means (500; 600) so that, when said inhalation-sensitive member (60) is deformed and/or moved under the effect of inhaling, it enables said dispenser means (200) to be actuated, **characterized in that** said device comprises a protective element (1) of said inhalation-controlled trigger system, said protective element (1) being disposed between said mouthpiece (400) and said inhalation-sensitive member (60), said protective element (1) being made of a porous material that allows air to pass through it but blocks the passage of water, dust and foreign bodies.

2. The device according to claim 1, wherein said protective element (1) is made of an open-pore sintered material.

3. The device according to any one of claims 2, wherein said open-pore sintered material comprises a material of the polyolefin type, such as polyethylene (PE), polypropylene (PP) or a mixture thereof, and/or of the polytetrafluoroethylene (PTFE) type.

4. The device according to any one of the preceding claims, wherein said protective element (1) is subjected to an antibacterial treatment.

5. The device according to any one of the preceding claims, wherein said product reservoir (100) containing a fluid product and a propellant gas is mounted to slide axially relative to said body (10), a metering valve (200), including a valve member (210), being assembled on said reservoir (100) for selectively dispensing the fluid product.

6. The device according to claim 5, comprising a blocking element (500) that is movable and/or deformable between a blocking position in which said metering valve (200) cannot be actuated, and an actuation position in which said metering valve (200) can be actuated, a trigger element (600) that is movable and/or deformable between a locking position in which it blocks said blocking element (500) in its blocking position, and a release position in which it does not block said blocking element (500), and said inhalation-sensitive member (60) co-operating with said trigger element (600), so that when said inhalation-sensitive member (60) is deformed and/or moved, it moves and/or deforms said trigger element (600) towards its release position, thereby making it possible to move and/or deform said blocking element (500) from its blocking position towards its actuation position.

7. The device according to any one of the preceding claims, wherein an actuator member (800) is mounted to move axially, in particular in sliding, in said body (10) between a rest position and a primed position, a spring (850) being arranged between said actuator member (800) and said reservoir (100) or an element (900; 950) that is secured to said reservoir (100), so that when said actuator member (800) moves towards its primed position, said spring (850) is compressed, so as to transmit an axial force (F) to said reservoir (100).

8. The device according to claim 7, wherein a laterally-actuated pusher (20) is mounted to move in pivoting and/or in translation on said body (10) between a rest position and a working position, a movement of said laterally-actuated pusher (20) towards its working position moving said actuator member (800) axially towards its primed position.

9. The device according to any one of the preceding claims, wherein said inhalation-sensitive member (60) includes a deformable membrane that defines a deformable air chamber, said deformable membrane being fastened to said trigger element (600), said deformable membrane being deformed during inhaling.
